# EUROPEAN PATENT APPLICATION

(11) **EP 4 226 882 A1**
(43) Date of publication of application: **16.08.2023**
(21) Application number: 23180908.8
(22) Date of filing: 02.07.2020
(51) Int. Cl.: A61B 18/14, A61B 18/00, A61B 17/00, A61B 34/20

(54) **REDUCED IMPEDANCE ELECTRODE DESIGN**

(30) Priority: 02.07.2019 US 201962869779 P
(62) Divisional of application: 20747107.9
(71) Applicant: St. Jude Medical, Cardiology Division, Inc., St. Paul, MN 55117 (US)
(72) Inventor: MANDA, Rishi, Edina, Minnesota 55436 (US); PEDERSON, Brian, Little Falls, Minnesota 56345 (US); OLSON, Gregory K., Elk River, Minnesota 55330 (US)
(74) Representative: Kramer Barske Schmidtchen Patentanwälte PartG mbB

(57) **Abstract**

A catheter for electrophysiological measurement can include a catheter shaft including a proximal end and a distal end. An electrical conductor can be attached to the catheter. In some examples, a catheter tip portion can be located at the distal end of the catheter shaft. The catheter tip portion can include an electrical conductor and an electrode. In some examples, the electrical conductor can be a trace along a flexible circuit. The electrical conductor can be configured to be communicatively coupled to an ECU, and the electrode can be disposed on the electrical conductor. In various examples, a contact surface area of the electrode can include an impedance reduction layer.

## Description

### BACKGROUND

### a. Field

The instant disclosure relates to catheters, such as catheters for diagnosis or treatment of various medical conditions.

### b. Background Art

Catheters have been used for cardiac medical procedures for many years. Catheters can be used, for example, to diagnose and treat cardiac arrhythmias, while positioned at a specific location within a body that is otherwise inaccessible without a more invasive procedure. In general, mapping catheters can be used for diagnosing electrophysiology and generating three-dimensional models of tissue within the body. Other catheters, such as ablation catheters can be used for treatment of some cardiac arrhythmias. Some catheters are configured to perform both mapping and ablation functions. In relation to mapping catheters, a tip portion of the catheter often has one or more electrodes for measuring electrophysiological signals (e.g., biosignals) within tissue. Various configurations of mapping catheters exist. Some mapping catheters have a single electrode for performing electrophysiological measurements whereas other mapping catheters can include a plurality of electrodes, such as an array of electrodes for collecting simultaneous measurements at various locations along the tissue. It can be desirable to increase the number of electrodes on the catheter in order to collect a greater amount of measurement data. In some instances, the collection of simultaneous data can also be advantageous for mapping and diagnosis purposes.

Because of geometrical confines within the body, both the placement of electrodes and use of an increased number of electrodes on a catheter can create challenges. Reducing the size and spacing of the electrodes can provide for an increase in the number and density of electrodes on the catheter. However, reducing the size of the electrodes correspondingly reduces the surface area of the electrodes for collecting electrophysiological measurement. As the size of the electrodes decreases, the electrical performance of the electrodes can be affected in some instances. The foregoing discussion is intended only to illustrate the present field and should not be taken as a disavowal of claim scope.

### BRIEF SUMMARY

The instant disclosure relates to high-density mapping catheter tips and to map-ablate catheter tips for diagnosing and treating cardiac arrhythmias via, for example, radio-frequency (RF) ablation. In particular, the instant disclosure relates to flexible high-density mapping catheter tips, and to flexible ablation catheter tips that can also have onboard high-density mapping electrodes. In some embodiments, the catheters may include irrigation.

In an example, a catheter for electrophysiological measurement can include a catheter shaft including a proximal end and a distal end. An electrical conductor attached to the catheter. In an example, a catheter tip portion can be located at the distal end of the catheter shaft. The catheter tip portion can include an electrical conductor and an electrode. In some examples, the electrical conductor can be a trace along a flexible circuit. The electrical conductor can be configured to be communicatively coupled to an electronic control unit (ECU). The electrode can be disposed on the electrical conductor. A contact surface area of the electrode can include an impedance reduction layer.

In various examples, the impedance reduction layer can be a platinum coating or a platinum iridium coating. In some instances, the thickness of the impedance reduction layer can be between 1-5 µm or 1-30 µm (inclusively). The contact surface area of the electrode or impedance reduction layer can include, but is not limited to, 1.0 mm², 0.5 mm², or less. In an example, the impedance reduction layer can be configured to reduce the impedance at the electrode by at least fifty percent as compared to a similarly sized electrode without the impedance reduction layer. In another example, the impedance reduction layer can be configured to increase the biocompatibility of the electrode, for instance, as compared to an electrode including a contact surface area coated with gold. An intermediate layer (e.g., a gold layer) can be disposed between a copper layer of the electrical conductor and the impedance reduction layer. In a further example, the impedance reduction layer can include a surface texture having a surface roughness, such as a surface roughness between 1 and 30 µm.

In another example, a method of making a catheter including a reduced impedance electrode can include attaching an electrical conductor to the catheter, such as attaching the electrical conductor to a catheter tip portion located at a distal end of a catheter. The electrical conductor can be configured to be communicatively coupled to an electronic control unit (ECU). In a further example, the method can include disposing an impedance reduction layer on the electrical conductor. In an example, the method can include attaching a flexible circuit to a catheter, wherein the flexible circuit includes the electrical conductor and the impedance reduction layer. The electrical conductor can be disposed along a dielectric layer of the flexible circuit, and the impedance reduction layer can be disposed on the electrical conductor. In further examples, attaching the flexible circuit to the catheter can include attaching the flexible circuit to a spine of a basket catheter.

In an example, the impedance reduction layer can include a contact surface area configured as an electrode to make contact with tissue. In a further example, the impedance reduction layer can include a material selected from a group comprising platinum or platinum iridium.

In an example, the impedance reduction layer can include a thickness between 1-5 µm or 1 to 30 µm (inclusively). In some examples, the size of the contact surface area can include, but is not limited to, less than 1.0 mm² or less than 0.5 mm². In various examples, the flexible circuit can include a gold layer between a copper layer of the electrical conductor and the impedance reduction layer. In some instances, the impedance reduction layer can include a surface texture. For example, the surface texture can include a surface roughness between 1 and 30µm. The surface texture can be applied by various means, such as formed using a laser.

The foregoing and other aspects, features, details, utilities, and advantages of the present disclosure will be apparent from reading the following description and claims, and from reviewing the accompanying drawings.

### BRIEF DESCRIPTION OF THE DRAWINGS

**FIG. 1** illustrates one example of a system for navigating and operating a medical device within a body.
**FIG. 2** depicts an example of one or more electrodes along at least one spine of a basket catheter.
**FIG. 3** illustrates an example of a flattened view of a flexible circuit including one or more electrodes.
**FIG. 4** depicts an example of a partial cross section of a catheter including at least one electrode with an impedance reduction layer.
**FIG. 5** illustrates another example of a partial cross section of a catheter including at least one electrode with an impedance reduction layer.
**FIG. 6** illustrates an example of a tip portion of a catheter including a flexible array of small-scale electrodes.
**FIG. 7** depicts an example of an impedance reduction layer including a surface texture.
**FIG. 8** illustrates another example of an impedance reduction layer including a surface texture.
**FIG. 9** illustrates a further example of an impedance reduction layer including a surface texture.
**FIG. 10** depicts an example of an ablation catheter including a flexible circuit with a plurality of electrodes having an impedance reduction layer.
**FIG. 11** is a chart depicting impedance measurements of examples of various electrodes.

### ASPECTS

1. A catheter for electrophysiological measurement, the catheter comprising:
   a catheter shaft including a proximal end and a distal end;
   an electrical conductor attached to the catheter and configured to be communicatively coupled to an electronic control unit (ECU); and
   an electrode disposed on the electrical conductor, wherein a contact surface area of the electrode includes an impedance reduction layer.
2. The catheter of aspect 1, wherein the electrical conductor is a trace positioned along a flexible circuit.
3. The catheter of aspect 1, wherein the impedance reduction layer is a platinum coating.
4. The catheter of aspect 3, wherein the platinum coating is a platinum iridium coating.
5. The catheter of aspect 1, further comprising a gold layer disposed between a copper layer of the electrical conductor and the impedance reduction layer.
6. The catheter of aspect 5, wherein the impedance reduction layer is configured to reduce the impedance at the electrode by at least fifty percent as compared to a similarly sized electrode without the impedance reduction layer.
7. The catheter of aspect 1, wherein the impedance reduction layer has a thickness of between 1 µm and 30 µm.
8. The catheter of aspect 1, wherein the impedance reduction layer is configured to increase the biocompatibility of the electrode.
9. The catheter of aspect 1, wherein the contact surface area of the impedance reduction layer is 1.0 mm² or less.
10. The catheter of aspect 1, wherein the contact surface area of the impedance reduction layer is 0.5 mm² or less.
11. The catheter of aspect 1, wherein the impedance reduction layer includes a surface texture having a surface roughness between 1 and 30µm.
12. A method of making a catheter including a reduced impedance electrode, the method comprising:
   attaching an electrical conductor to the catheter, wherein the electrical conductor is configured to be communicatively coupled to an electronic control unit (ECU); and
   disposing an impedance reduction layer on the electrical conductor, the impedance reduction layer including a contact surface area configured as an electrode for making contact with tissue, wherein the impedance reduction layer includes a material selected from a group comprising platinum or platinum iridium.
13. The method of aspect 12, wherein the flexible circuit includes a gold layer positioned between a copper layer of the electrical conductor and the impedance reduction layer.
14. The method of aspect 12, wherein the impedance reduction layer has a thickness of between 1 µm and 30 µm.
15. The method of aspect 12, wherein the contact surface area is 1.0 mm² or less.
16. The method of aspect 12, further comprising applying a surface texture to the impedance reduction layer.
17. The method of aspect 16, wherein the surface texture includes a surface roughness between 1 and 30µm.
18. The method of aspect 16, further comprising applying the surface texture using a laser.
19. The method of aspect 12, wherein attaching the electrical conductor to the catheter further comprises attaching a flexible circuit including the electrical conductor to the catheter.
20. The method of aspect 12, wherein attaching the electrical conductor to the catheter includes attaching the electrical conductor to a spine of a basket catheter.

### DETAILED DESCRIPTION OF EMBODIMENTS

Several embodiments of an electrode, such as an electrode for a flexible, high-density mapping catheter and map-ablate catheters as well as ablation therapy catheters are disclosed herein. In general, tip portions of these various catheters comprise one or more electrodes for measuring electrophysiological signals of a patient or for delivering ablation therapy to tissue of the patient. Reducing the size and spacing of the electrodes can provide for an increase in the number and density of electrodes along the tip portion, can allow for a reduction in the size of the tip portion itself, and can reduce the cost of fabrication. As the size of the electrodes is reduced, however, the impedance between the electrode and the respective tissue in contact with the electrode can be increased due to the reduction in surface area of the electrode. The present inventors have recognized that an electrode having an impedance reduction coating, such as platinum or platinum iridium (PtIr) surface coating, can reduce the impedance between the electrode and tissue contacting the electrode. Details of the various embodiments of the present disclosure are described below with specific reference to the figures.

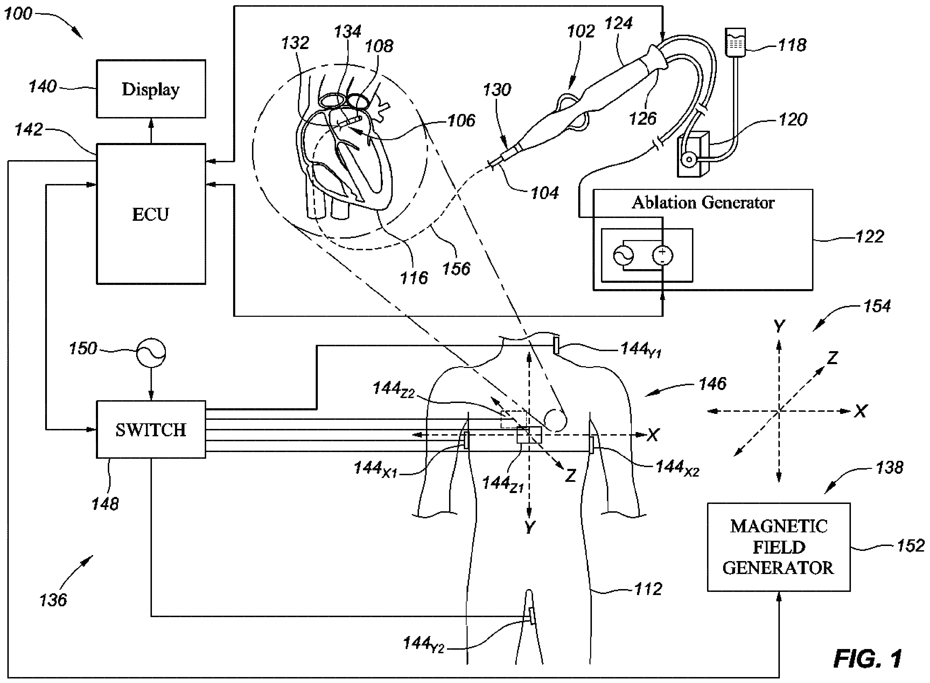

**FIG. 1** illustrates one example of a system 100 for navigating and operating a medical device within a body 112. In the illustrated example, the medical device comprises a catheter 102, such as an ablation catheter, that is shown schematically entering a heart (e.g., tissue 116) that has been exploded away from the body 112. The catheter 102, in this example, is depicted as a mapping catheter. It should be understood, however, that the system 100 can find application in connection with a wide variety of medical devices used within the body 112 for diagnosis or treatment. Further, it should be understood that the system 100 can be used to navigate medical devices used in the diagnosis or treatment of portions of the body 112 other than cardiac tissue 116. Further description of the systems and components are contained in U.S. patent application 13/839,963 filed on 15 March 2013, which is hereby incorporated by reference in its entirety as though fully set forth herein.

The catheter 102 can include a handle 124, a cable connector or interface 126 at a proximal end of the handle 124, and a shaft 104 (also referred to herein as a catheter shaft). The shaft 104 can include a proximal end 130, a distal end 132. A tip portion 106 can be located at the distal end 132. The handle 124 provides a location for the physician to hold the catheter 102 and can further provide means for steering or guiding the shaft 104 within the body 112. For example, the handle 124 can include means to change the length of one or more pull wires extending through the catheter 102 from the handle 124 to the distal end 132 of shaft 104. The construction of the handle 124 can vary.

The shaft 104 can be made from conventional materials such as polyurethane and can define one or more lumens configured to house and/or transport electrical conductors 156, fluids, or surgical tools. The shaft 104 can be introduced into a blood vessel or other structure within the body 112 through a conventional introducer. The shaft 104 can then be steered or guided through the body 112 to a desired location such as the tissue 116 using guide wires or pull wires or other means known in the art including remote control guidance systems. The shaft 104 can also permit transport, delivery, and/or removal of fluids (including irrigation fluids and bodily fluids), medicines, and/or surgical tools or instruments. It should be noted that any number of methods can be used to introduce the shaft 104 to areas within the body 112. This can include introducers, sheaths, guide sheaths, guide members, guide wires, or other similar devices. For ease of discussion, the term introducer will be used throughout.

In some examples, the system 100 can include a positioning system, a display 140, and an electronic control unit (ECU) 142. The ECU 142 can include, but is not limited to, a central processing unit (CPU), graphics processing unit (GPU), microprocessor, application specific integrated circuit (ASIC), a field programmable gate array (FPGA), complementary metal-oxide-semiconductor (CMOS), or the like. In some examples, the ECU can include memory, such as random-access memory (RAM), read-only memory (ROM), programmable read-only memory (PROM), erasable programmable read-only memory (EPROM), and electrically erasable programmable read-only memory (EEPROM), dynamic random-access memory (DRAM), static random-access memory (SRAM), Flash memory, or the like.

The positioning system, such as the electric-field-based positioning system 136 or the magnetic-field-based positioning system 138, is provided to determine the position and orientation of the catheter 102, the tip portion 106, and similar devices within the body 112. For instance, the location or orientation of the tip portion 106 can be based on a fiducial or location of one or more electrodes of the tip portion 106, such as locational electrodes 134. In some examples, the locational electrodes 134 can include ring electrodes as shown in the example of FIG. 1. The system 136 can comprise, for example, the EnSite^{™} NavX^{™} system sold by Abbott Laboratories of St. Paul, Minnesota, and described in, for example, U.S. Patent No. 7,263,397 titled "Method and Apparatus for Catheter Navigation and Location Mapping in the Heart," the entire disclosure of which is hereby incorporated by reference as though fully set forth herein. The systems 136 and 138 can comprise, for example, the EnSite Precision^{™} system sold by Abbott Laboratories of St. Paul, Minnesota. The system 136 operates based upon the principle that when low amplitude electrical signals are passed through the thorax, the body 112 acts as a voltage divider (or potentiometer or rheostat) such that the electrical potential or field strength measured at one or more electrodes, such as locational electrodes 134, on the catheter 102 can be used to determine the position of the catheter 102 relative to a pair of external patch electrodes using Ohm's law and the relative location of a reference electrode (e.g., in the coronary sinus).

In the configuration shown in FIG. 1, the electric-field-based positioning system 136 further includes three pairs of patch electrodes 144, which are provided to generate electrical signals used in determining the position of the catheter 102 within a three-dimensional coordinate system 146. The patch electrodes 144 can also be used to generate electrophysiology (EP) data (e.g., electrophysiological signals) regarding the tissue 116. To create axes-specific electric fields within body 112, the patch electrodes are placed on opposed surfaces of the body 112 (e.g., chest and back, left and right sides of the thorax, and neck and leg) and form generally orthogonal X, Y, and Z axes. A reference electrode is typically placed near the stomach and provides a reference value and acts as the origin of the coordinate system 146 for the navigation system.

In accordance with this exemplary electric-field-based positioning system 136 as depicted in FIG. 1, the patch electrodes include right side patch 144_{X1}, left side patch 144_{X2}, neck patch 144_{Y1}, leg patch 144_{Y2}, chest patch 144zi, and back patch 144_{Z2}; and each patch electrode is connected to a switch 148 (e.g., a multiplex switch) and a signal generator 150. The patch electrodes 144_{X1}, 144_{X2} are placed along a first (X) axis; the patch electrodes 144_{Y1}, 144_{Y2} are placed along a second (Y) axis, and the patch electrodes 144zi, 144_{Z2} are placed along a third (Z) axis. Sinusoidal currents are driven through each pair of patch electrodes, and voltage measurements for one or more position sensors (e.g., locational electrodes 134) associated with the catheter 102 are obtained. The measured voltages are a function of the distance of the position sensors from the patch electrodes. The measured voltages are compared to the potential at the reference electrode and a position of the position sensors within the coordinate system 146 of the navigation system is determined.

The magnetic-field-based positioning system 138 in this example employs magnetic fields to detect the position and orientation of the catheter 102 within the body 112. The system 138 can include the GMPS system made available by MediGuide, Ltd. and generally shown and described in, for example, U.S. Patent No. 7,386,339 titled "Medical Imaging and Navigation System," the entire disclosure of which is hereby incorporated by reference as though fully set forth herein. In such a system, a magnetic field generator 152 can be employed having three orthogonally arranged coils (not shown) to create a magnetic field within the body 112 and to control the strength, orientation, and frequency of the field. The magnetic field generator 152 can be located above or below the patient (e.g., under a patient table) or in another appropriate location. Magnetic fields are generated by the coils and current or voltage measurements for one or more position sensors associated with the catheter 102 are obtained. The measured currents or voltages are proportional to the distance of the sensors from the coils, thereby allowing determination of a position of the sensors within a coordinate system 154 of system 138.

As the catheter 102 moves within the body 112, and within the electric field generated by the electric-field-based positioning system 136, the voltage readings from the locational electrodes 134 change, thereby indicating the location of catheter 102 within the electric field and within the coordinate system 146 established by the system 136. The locational electrodes 134 can be adapted to communicate position signals to the ECU 142.

The catheter 102 can be configured to deliver treatment as well as geometric modeling or electrophysiological mapping. In some examples, the catheter 102 can include at least one electrode 108 configured to detect electrophysiological signals from the tissue or to provide energy for ablating the tissue. In an example, the electrode 108 can be communicatively coupled to the ablation generator 122 for delivery of electrical signals adapted to provide the ablation energy to the electrode 108. In some examples, the catheter 102 can be optionally connected to a fluid source 118 for delivering a biocompatible irrigation fluid such as saline through a pump 120. The pump 120 can include a fixed rate roller pump or variable volume syringe pump with a gravity feed supply from fluid source 118 as shown. The connector 126 provides mechanical, fluid, and electrical connections for conduits or cables extending from the pump 120 and the ablation generator 122. The catheter 102 can also include other conventional components not illustrated herein such as a temperature sensor, additional electrodes, and corresponding conductors or leads.

The ECU 142 provides a means for controlling the operation of various components of the system 100, including the catheter 102, the ablation generator 122, and magnetic generator 152 of the magnetic-field-based positioning system 138. The ECU 142 can also provide a means for determining electrophysiology characteristics (e.g., signals) of the tissue 116, the position and orientation of the catheter 102 relative to tissue 116 and the body 112, controlling the ablation of the tissue 116, or any combination thereof. The ECU 142 also provides a means for generating display signals used to control the display 140.

The display 140 is provided to convey information to a physician to assist in diagnosis and treatment. The display 140 can comprise one or more conventional computer monitors or other display devices. The display 140 can present a graphical user interface (GUI) to the physician. The GUI can include a variety of information including, for example, an image of the geometry of the tissue 116, electrophysiology data (e.g., maps of signals from the electrodes 108) associated with the tissue 116, graphs illustrating voltage levels over time for various locational electrodes 134, and images of the catheter 102 and other medical devices and related information indicative of the position of the catheter 102 and other devices relative to the tissue 116.

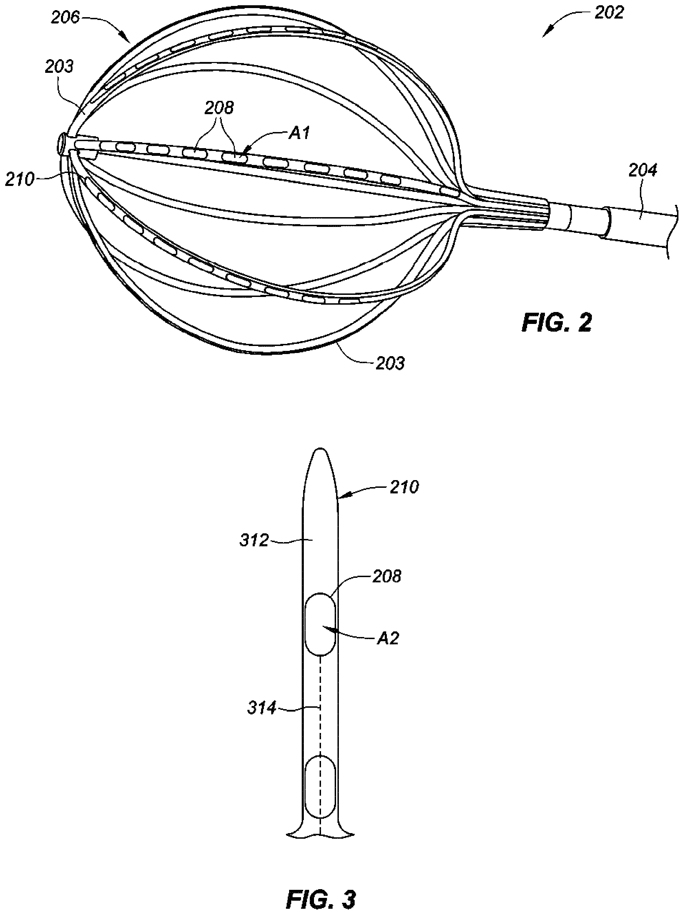

**FIG. 2** illustrates an example of a tip portion 206 of a catheter 202, such as a basket catheter, including one or more electrodes 208 along at least one spine 203 of the basket catheter. In the example of FIG. 2, the catheter 202 can be a mapping catheter that can be used to map electrophysiological signals within the heart of a patient, as previously described in FIG. 1. In various examples, the catheter 202 can be a mapping catheter, ablation catheter, a catheter for sensing electrophysiological signals, or other type of catheter.

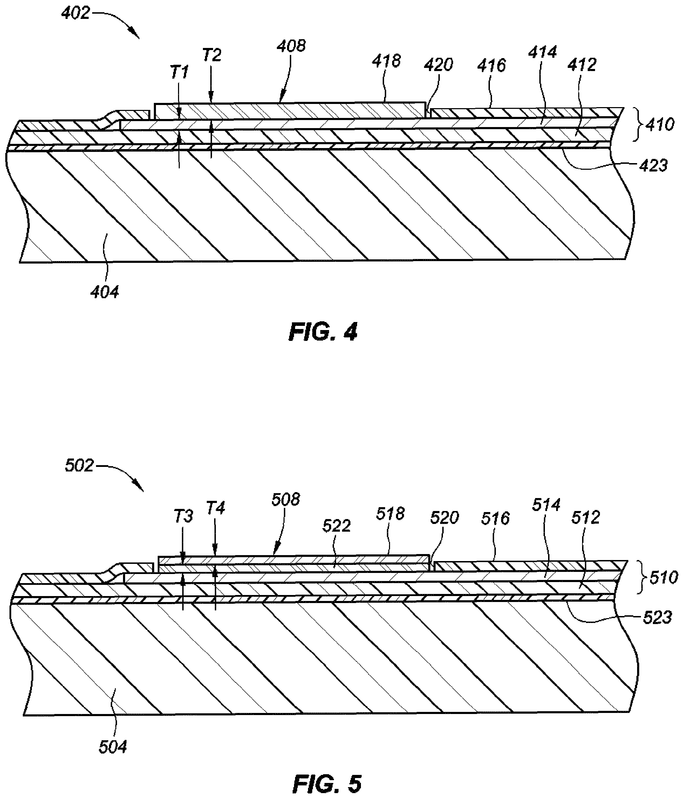

In the example of FIG. 2, the catheter 202 can include a plurality of electrodes 208 in a distributed pattern along the spine 203. In some examples, increasing the density of the electrodes 208 can provide for an increased number of electrodes 208 along the catheter 202 (e.g. the tip portion 206 of the catheter), an increased resolution of the measurement of the electrophysiological signals within (and accordingly an electrophysiological map) of the tissue, or a combination thereof. The density of the electrodes 208 can be increased by decreasing the spacing of the electrodes 208, decreasing a contact surface area *A1* of the electrodes 208, or both. Where the contact surface area *A1* of the electrodes 208 is decreased, the impedance between tissue and the electrodes 208 that are in contact with the tissue can increase accordingly. For instance, the impedance of the electrophysiological signal can include a resistance component and a reactance component. The resistance component and the reactance component can increase corresponding to the decrease in the contact surface area *A1* of the electrode 208. In another example, the reactance component can increase at a greater rate than the resistance component as the frequency of the electrophysiological signal decreases. In further example, the reactance component can increase at a greater rate than the resistance component as the contact surface area *A1* decreases. Accordingly, the reactance component can increase corresponding to the decrease in the contact surface area *A1,* a decrease of the frequency of the electrophysiological signal, or a combination thereof. In the example of FIGS. 2-10, the electrodes can be small-scale electrodes. As discussed herein, small-scale electrodes can include a contact surface area *A1* of less than one square millimeter. The electrodes 208 can include an impedance reduction coating (as shown in the examples of FIGS 4 and 5 and described further herein) to reduce the impedance of the electrodes, generally, and, in some examples, to mitigate the increased impedance of the reduced-size electrodes.

In some examples, the one or more electrodes 208 can be located on a flexible circuit 210. The flexible circuit 210 can be attached to a shaft 204 of the catheter 202. For instance, as shown in the example of FIG. 2, the flexible circuit 210 can include a complimentary shape to conform the flexible circuit 210 to the geometry of the shaft 204.

**FIG. 3** depicts an example of a flattened detail view of the flexible circuit 210, such as the flexible circuit 210 shown in the example of FIG. 2. The flexible circuit 210 can include a plurality of electrodes 208, such as the electrodes shown and described further herein. For instance, the flexible circuit 210 can include a dielectric layer 312, the electrodes 208, and a plurality of electrical conductors 314. Further examples of the flexible circuit, dielectric layer, electrodes, and electrical conductors are shown and described in the examples of FIGS. 4 and 5. The electrical conductors can communicatively couple the respective electrodes 208 to the ECU (as shown in FIG. 1 and discussed herein). The dielectric layer 312 can include an electrically insulating polymer, such as polyimide, polyester, polyethylene terephthalate, polyethylene naphthalate, polyetherimide, various fluropolymers, copolymers, or other suitable flexible substrate. The electrodes 208 can be located along a first side of the dielectric layer 312. In an example, an adhesive (shown in the example of FIGS. 4 and 5) can attach the second side of the dielectric layer to the spine 203 (as shown in the example of FIG. 2), a substrate of the catheter 302, or a shaft of the catheter 302. In other examples, the flexible circuit 310 can be attached to the catheter 302 by various other means, such as heat staking, insert molding, additive manufacturing, ultrasonic welding, mechanically fastening, or the like.

In the example of FIG. 3, the shape (outer profile) of the flexible circuit 210 configured to conform to the geometry of the catheter. The flexible circuit 210 can be of any shape and size that can be utilized for electrophysiology, ablation therapy, or other medical diagnosis or treatment. The electrodes 208 can be arranged along the flexible circuit 210 in a variety of patters, such as staggered, circular, grid, diamond, or other pattern. In an example, the contact surface area *A2* of the electrodes 208 can be various shapes, such as round, ovoid (as shown in the examples of FIGS. 2 and 3), ring, rectangular, elongate, or other shape. As shown in FIG. 3, the shape of the flexible circuit 210 can accommodate the pattern of the electrodes 208 as located along the flexible circuit 210. In some examples, the electrodes, such as the electrodes 208, can include an impedance reduction layer as shown in FIG. 4 and described herein. In other examples, the electrodes can include an impedance reduction layer and an intermediate layer as shown in the example of FIG. 5 and described herein.

**FIG. 4** depicts an example of a partial cross section of a catheter 402 including at least one electrode 408 disposed thereon. In the example of FIG. 4, the electrode 408 includes an impedance reduction layer 418. As depicted, the electrode 408 can be included in a flexible circuit 410. The flexible circuit 410 can be attached to a substrate 404 of the catheter 402, such as the spine 203 or a catheter shaft 204 (as shown in the example of FIGS. 2-3). In an example the an adhesive 423 can be located between the dielectric layer 412 and the substrate 404 to attach the flexible circuit 410 to the substrate 404. The flexible circuit 410 can be a single layer flexible circuit as shown in the example of FIG. 4 or a multi-layer flexible circuit. In the example FIG. 4, the flexible circuit 410 can include a first dielectric layer 412, an electrical conductor 414, a second dielectric layer 416, and the electrode 408 including the impedance reduction layer 418. As previously discussed, the dielectric layer, such as the dielectric layer 412 or the dielectric layer 416, can include an electrically insulating polymer, for example polyimide, polyester, Polyethylene terephthalate, polyethylene naphthalate, polyetherimide, various fluropolymers, copolymers, or other suitable flexible substrate. An electrically conductive layer can be disposed on the first dielectric layer 412 to form the electrical conductor 414. The electrical conductor 414 can be constructed from a material including, but not limited to, copper, copper nickel alloy, inconel, silver filled epoxy, carbon, aluminum, gold, silver, platinum, or the like. In various examples, the electrical conductor 414 can be a metallic film that is applied to the first dielectric layer 412 with an adhesive, or the electrical conductor 414 can be electrodeposited on to the dielectric layer 142. In an example, the conductive layer can be etched to form the electrical conductor 414, for example to form the geometry of the electrical traces for routing electrical signals between the electrode 408 and the ECU. The electrical conductor 414 can include a thickness *T1* between 10 µm and 125 µm, preferably between 125 µm and 25 µm (inclusively).

In a further example, the electrical conductor 414 can be directly attached to the substrate 404 of the catheter 402, such as a spine (e.g., spine 203), a shaft (e.g., shaft 104), or tip portion (e.g., tip portion 206) of the catheter 402. In an example, the electrical conductor 414 can be a metallic film that is applied to the substrate 404 with an adhesive. In various other examples, the electrical conductor 414 can be applied to the substrate 404 using electrodeposition, aerosol jet, vapor deposition, chemical deposition, or the like.

The impedance reduction layer 418 can be disposed on the electrical conductor 414. The impedance reduction layer 418 can reduce impedance between the tissue and the electrical conductor 414. As discussed further herein, the impedance reduction layer 418 can include platinum (Pt), platinum iridium (PtIr), or the like. Where the impedance reduction layer 418 includes platinum or platinum iridium, the impedance reduction layer 418 can increase the biocompatibility of the electrode as compared to electrodes including a gold plated contact surface area. In some examples, the impedance reduction layer 418 can be a surface texture or can be combined with a surface texture configured to reduce the impedance between the tissue and the electrode 408. The impedance reduction layer 418 can be disposed on the electrical conductor 414, for instance, by one or more means including, but not limited to, electrodeposition, vapor deposition, chemical deposition, aerosol jet, application of a foil, or other type method of applying the impedance reduction layer. The impedance reduction layer 418 can include a thickness T2 between 1 µm and 30 µm, preferably between 1 µm and 5 µm (inclusively). In a further example, the electrical conductor 414 can be directly attached to the substrate 404 of the catheter 402, such as or a spine (e.g., spine 203), a shaft (e.g., shaft 104), or tip portion (e.g., tip portion 206) of the catheter 402.

The second dielectric layer 416 can be disposed over the electrical conductor 414 to electrically insulate and protect the electrical conductor 414. The second dielectric layer 416 can include one or more apertures 420 to expose the electrode 408 to provide a contact surface area for placement against tissue within the body. In some examples, the aperture 420 can be constructed by laser cutting, punching, etching, or the like. In the example of FIG. 4, the contact surface area of the electrode 408 can be extended through the aperture 420. In other examples, the second dielectric layer 416 can be disposed over a portion of the electrode 408 (e.g., the impedance reduction layer 418) as well as the electrical conductor 414. Accordingly, the second dielectric layer 416 can increase the attachment strength of the electrode 408 to the flexible circuit 410, such as to the electrical conductor 414, first dielectric layer 412, or a combination thereof. The first dielectric layer 412, the second dielectric layer 416, or both can include a thickness of between 25 µm to 125 µm, preferably between 25 µm and 50 µm (inclusively).

**FIG. 5** illustrates an example of a partial cross section of a catheter 502 including at least one electrode 508 disposed thereon. In the example of FIG. 5, the electrode 508 includes an impedance reduction layer 518 and an intermediate layer 522. In an example, an electrical conductor 514 can be directly attached to a substrate 504 of the catheter 502, such as or a spine (e.g., spine 203) or a shaft (e.g., shaft 104) of the catheter 502. The electrode 508 can be electrically coupled to the electrical conductor 514, for instance, disposed on the electrical conductor 514. One or more means of disposing the electrical conductor 514 on the substrate 504 (e.g., a catheter tip portion, spine, or shaft) can include, but are not limited to, electrodeposition, vapor deposition, chemical deposition, aerosol jet, application of a foil, or other method of applying an intermediate metallic layer.

In a further example, a flexible circuit 510 can be attached to the catheter 502, such as the shaft, spine, or arm of the catheter 502. The flexible circuit 510 can be a single layer flexible circuit or a multi-layer flexible circuit as discussed herein. As shown in FIG. 5, the flexible circuit 510 can include a first dielectric layer 512, the electrical conductor 514, a second dielectric layer 516, and the electrode 508 (including the impedance reduction layer 518 and the intermediate layer 522). As previously discussed, the dielectric layer, such as the dielectric layer 512 or the dielectric layer 516, can include an electrically insulating polymer, for example polyimide, polyester, Polyethylene terephthalate, polyethylene naphthalate, polyetherimide, various fluropolymers, copolymers, or other suitable flexible substrate. In an example the an adhesive 523 can be located between the dielectric layer 512 and the substrate 504 to attach the flexible circuit 510 to the substrate 504. An electrically conductive layer can be disposed on the first dielectric layer 512 to form the electrical conductor 514, and the second dielectric layer 516 can be disposed over the electrical conductor 514 to electrically insulate and protect the electrical conductor 514.

The intermediate layer 522 can be disposed on the electrical conductor 514, and the impedance reduction layer 518 can be disposed on the intermediate layer 522. Accordingly, the intermediate layer 522 can be disposed between the electrical conductor 514 and the impedance reduction layer 518. In an example, the intermediate layer 522 can include a material that is compatible with the material of the electrical conductor 514 and the impedance reduction layer 518. For instance, the intermediate layer 522 can facilitate adhesion between the electrical conductor 514 and the impedance reduction layer 518. In an example, the intermediate layer 522 can include gold or a gold alloy. In a further example, the intermediate layer 522 can include electroless nickel immersion gold (ENIG) or electroless nickel electroless palladium immersion gold (ENEPIG). In a further example, the intermediate layer can include a platinum (Pt) layer and the impedance reduction layer can include a platinum iridium (PtIr) layer. One or more means of disposing the intermediate layer 522 on the electrical conductor 514 can include, but are not limited to, electrodeposition, vapor deposition, chemical deposition, aerosol jet, application of a foil, or other method of applying an intermediate metallic layer. In some examples, the intermediate layer 522 can include a thickness *T3* between 1 µm and 25 µm (inclusively), preferably between 5 µm and 15 µm (inclusively), or more preferably 10 µm.

The impedance reduction layer 518 can reduce impedance between the tissue and electrical conductor 514. As discussed herein, the impedance reduction layer 518 can include platinum (Pt), platinum iridium (PtIr), or the like. In some examples, the impedance reduction layer 518 can be a surface texture or can be combined with a surface texture configured to reduce the impedance between the tissue and the electrode 508. The impedance reduction layer 518 can be deposited on the intermediate layer 522, for instance, by one or more means including, but not limited to, electrodeposition, vapor deposition, chemical deposition, aerosol jet, application of a foil (including an impedance reducing material as described herein), or other method of applying the impedance reduction layer 518. The impedance reduction layer 518 can include a thickness *T4* between 1 µm and 30 µm or preferably between 1 µm and 5 µm (inclusively).

The second dielectric layer 516 can be disposed over the electrical conductor 514 to insulate and protect the electrical conductor 514. The second dielectric layer 516 can include one or more apertures 520 to expose the electrode 508 to provide a contact surface area for placement against tissue within the body. In some examples, the aperture 520 can be constructed by laser cutting, punching, etching, or the like. In the example of FIG. 5, the contact surface area of the electrode 508 can be extended through the aperture 520. In other examples, the second dielectric layer 516 can be disposed over a portion of the electrode 508 (e.g., the impedance reduction layer 518) as well as the electrical conductor 514. Accordingly, the second dielectric layer 516 can increase the attachment strength of the electrode 508 to the flexible circuit 510, such as to the electrical conductor 514, first dielectric layer 512, or a combination thereof. The first dielectric layer 512, the second dielectric layer 516, or both can include a thickness of between 25 µm to 125 µm, preferably between 25 µm and 50 µm (inclusively).

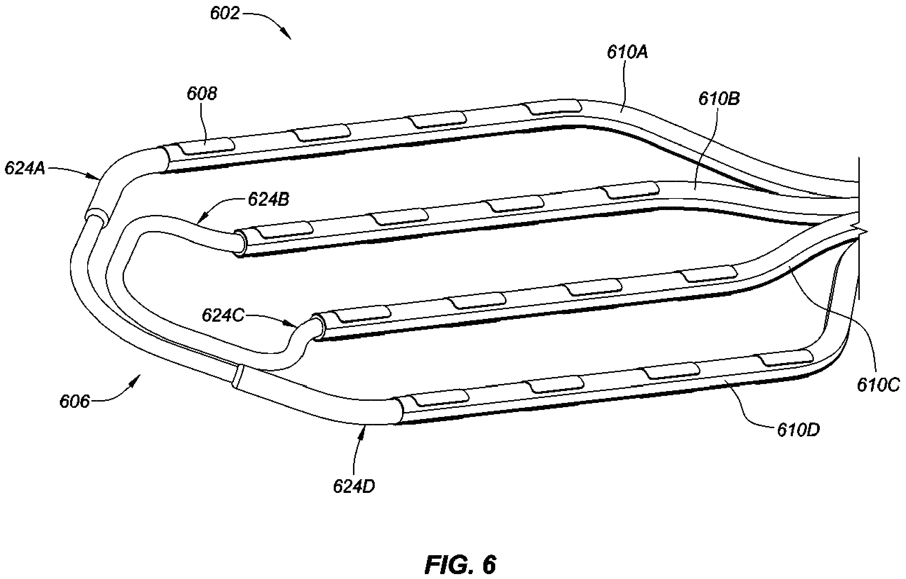

**FIG. 6** illustrates an example of a tip portion 606 of a catheter 602 including a flexible array of small-scale electrodes. In the example of FIG. 6, the array is a planar array (or 'paddle' configuration) of small-scale electrodes including four side-by-side, longitudinally-extending arms 624A-D forming the flexible framework along which a plurality of electrodes are coupled. For instance, in an example, the array can include thirty-two 1.0 mm long x 0.8 mm diameter ring electrodes 608. In other examples, the electrodes 608 can include a contact surface area less than 1.0 mm², such as 0.5 mm², or a contact surface area between 1.0 mm² and 0.3 mm². As shown in FIG. 6, the arms can include a first outboard arm 624A, a second outboard arm 624D, a first inboard arm 624B, and a second inboard arm 624C. These arms 624A-D are laterally separated from each other (e.g., by approximately 3.3 mm). Each of the four arms 624A-D carries eight electrodes 608, spaced along the length of the respective arms 624A-D. In the depicted example, these electrodes are longitudinally separated from each other by approximately 1.0 mm. Although FIG. 6 depicts four arms 624A-D, the array could comprise more or fewer arms.

In the example of FIG. 6, the electrodes 608 can be located on one or more flexible circuits, such as flexible circuits 610A-D. The flexible circuits 610A-D can include one or more of the features shown and described herein with respect to the flexible circuits 210-510 shown in the examples of FIGS. 2-5 and described herein. The flexible circuits 610A-D can be attached to the respective arms 624A-D. For instance, as shown in the example of FIG. 6, the flexible circuits 610A-D can include a complementary shape to conform the flexible circuits 610A-D to the geometry of the arms 624A-D.

In some examples, a few of these electrodes can be slightly longer. For instance, the most-distal electrode 608 on the first outboard arm 624A can be slightly enlarged as is the most-proximal electrode 608 on the second outboard arm 624D. These slightly enlarged electrodes can be used, for example, for more precise localization of the flexible array in mapping and navigation systems. It is also possible to drive ablation current between these enlarged electrodes, if desired, for bipolar ablation, or, alternatively to drive ablation current in unipolar mode between one or both of these enlarged electrodes and, for example a patch electrode located on a patient (e.g., on the patient's back). Similarly, the electrodes (on this or any of the other paddle catheters) can be used to perform unipolar or bipolar ablation. Alternatively or concurrently, current could travel between one or more of the enlarged electrodes and any one or all of the electrodes 608. This unipolar or bipolar ablation can create specific lines or patterns of lesions.

In further examples, the tip portion 606 of a catheter 602 can be combined with, the planner array and catheter tip portions of US Patent Publication US 2015/0374252 titled "FLEXIBLE HIGH-DENSITY MAPPING CATHETER TIPS AND FLEXIBLE ABLATION CATHETER TIPS WITH ONBOARD HIGH-DENSITY MAPPING ELECTRODES," the entire disclosure of which is hereby incorporated by reference as though fully set forth herein.

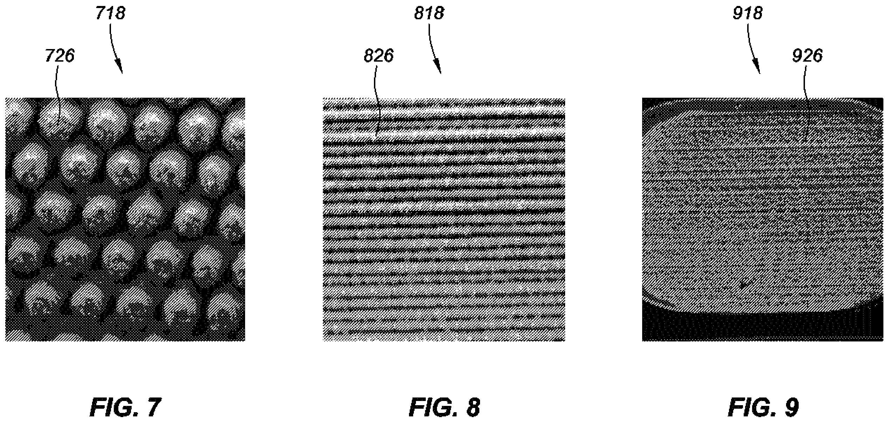

**FIGS. 7-9** illustrate examples of impedance reduction layers 718, 818, 918 including respective surface textures 726, 826, 926. The surface textures 726, 826, 926 can be applied to the contact surface area of the electrode. In some examples, the surface texture 726, 826, 926 can be applied to the impedance reduction layer of the electrode. The surface textures 726, 826, 926 can be configured to increase the contact surface area and accordingly reduce the impedance at the electrode. The surface texture can be used independently or in combination with the impedance reduction layer to reduce the impedance at the electrode.

The surface texture 726 includes a pattern of plumes having a height including, but not limited to, 1 to 30 µm. In another example, the surface texture 826 can include a pattern of lines including a depth or height between 1 and 30 µm. In a further example, the surface texture 926 can be a pattern of dimples including a depth between 1 and 30 µm.

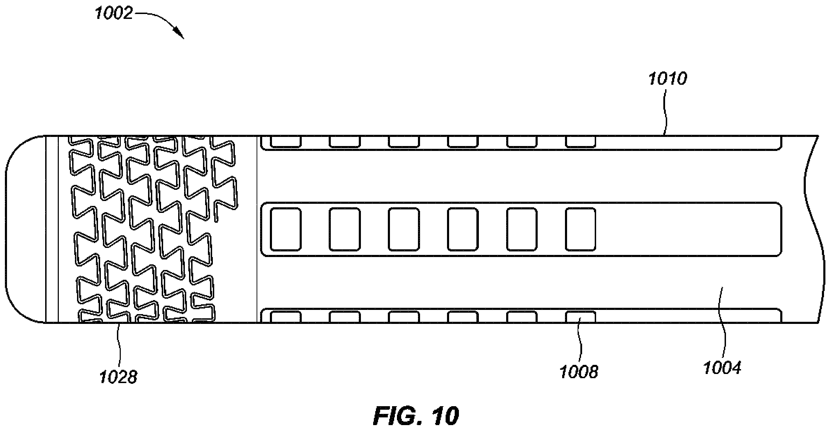

**FIG. 10** illustrates an example of a catheter 1002, such as an ablation catheter. The catheter 1002 can include one or more flexible circuits 1010 fixedly attached to the shaft 1004 of the catheter 1002. For example, the flexible circuits can be attached to the shaft 1004 using an adhesive. In the example of FIG. 10, the catheter 1002 can include four flexible circuits 1010. The flexible circuits can be arranged at 90 degrees radially from one another along an outer diameter of the shaft 1004. The flexible circuits 1010 can include a plurality of electrodes 1008 spaced thereon. The electrodes can include an impedance reduction layer as described further herein. In a further example, the flexible circuit 1010 can include an intermediate layer as previously described. The catheter 1002 can include an ablation electrode 1028 at the distal end of the shaft 1004. Accordingly, the ablation electrode can provide ablation therapy to the tissue and the electrodes 1008 can be used to measure electrophysiological signals within the tissue for diagnosis, mapping, or identification of an ablation site.

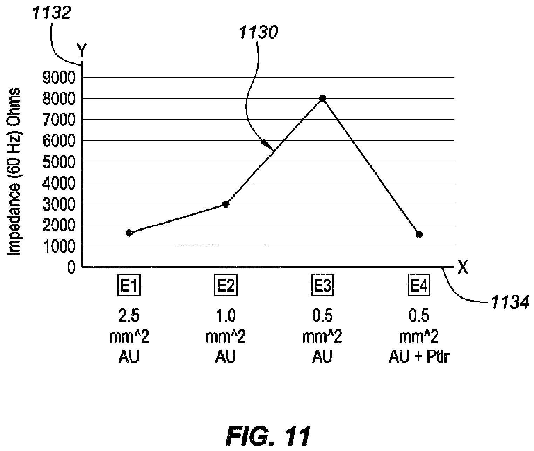

**FIG. 11** is a chart depicting an example of the impedance 1130 (Ohms) of various electrodes *E1, E2, E3,* and, *E4.* The chart includes impedance 1130 on the vertical axis 1132. The horizontal axis 1134 indicates the respective electrode samples *E1, E2, E3,* and *E4.* The example of *E1* can be an electrode including a contact surface area of 2.5 mm². For instance, *E1* can be a ring electrode in some examples. Electrode *E2* can be an example of an electrode including a contact surface area of 1.0 mm². In some instances, electrode *E2* can be a ring electrode or other type of electrode. In further examples, the electrodes *E1* and *E2* can be ring electrodes along an array similar to the array illustrated in FIG. 6. Electrode *E3* can be an electrode of a flexible circuit, such as a flexible circuit illustrated in any of the examples of FIGS. 2-6 or 10 as shown and described herein. The electrode *E3* can include a contact surface area of 0.5 mm². The respective electrodes *E1, E2,* and *E3* do not include an impedance reduction layer as described herein. For example, the electrodes *E1, E2,* and *E3* can include a gold layer over a copper electrical conductor, where the gold layer is exposed along the contact surface area.

The electrode *E4* includes a contact surface area of 0.5 mm². In an example, the electrode *E4* is disposed along a flexible circuit as shown and described herein. The electrode *E4* includes the impedance reduction layer as described herein, such as in the examples of FIGS. 2-9. As shown in the example of FIG. 11, the electrode *E4* includes a platinum iridium layer disposed over a gold intermediary layer.

The impedance 1130 at the electrodes *E1, E2,* and *E3* can increase as the contact surface area of the electrodes decreases (e.g., from 2.5 mm² to 0.5 mm² in the examples of electrodes *E1-E3*), as shown in FIG. 11. In some examples, the resistance component and the reactance component of the impedance 1130 can increase corresponding to the decrease in the contact surface area of the electrode. As previously mentioned, the impedance at the electrodes is dependent upon the frequency of the signals (e.g., electrophysiological signals). For instance, as the frequency of the electrophysiological signal decreases, the impedance can increase. As the frequency of the electrophysiological signal decreases, the reactance component can increase at a greater rate than the resistance component. Further, as the contact surface area decreases, the reactance component can increase at a greater rate than the resistance component. Accordingly, the share of the reactance component as a percentage of the impedance can increase corresponding to the decrease in the contact surface area, a decrease of the frequency of the electrophysiological signal, or a combination thereof.

In the example of FIG. 11, the electrode *E4* can be a reduced-size electrode and can include the impedance reduction layer as shown and described herein. As depicted in FIG. 11, the impedance at the electrode *E4* cane be reduced as compared to the impedance at electrode *E3* having the same contact surface area without the impedance reduction layer. As further shown in FIG. 11, the impedance at the electrode *E4* (including the impedance reduction layer) can be reduced as compared to the impedance of a larger electrode without the impedance reduction layer having a contact surface area of 1.0 mm² (e.g., electrode *E2*). Further, the impedance at the electrode *E4* can be similar to an electrode without the impedance reduction layer having a contact surface area of 2.5 mm² (e.g., electrode *E1*).

In an example, the resistance component, the reactance component, or both can be reduced by including the impedance reduction coating on the electrode. In another example, across a range of frequencies (e.g., 1 to 20,000 Hz) the resistance component, the reactance component, or combinations thereof can be reduced by inclusion of the impedance reduction layer. The impedance reduction layer can be used to reduce the impedance of the electrodes, generally, and in some examples, to mitigate the increased impedance of the reduced-size electrodes. Reducing the impedance at the electrodes can provide for increased fidelity of the electrophysiological signal measurement.

Although several embodiments have been described above with a certain degree of particularity, those skilled in the art could make numerous alterations to the disclosed embodiments without departing from the spirit of the present disclosure. It is intended that all matter contained in the above description or shown in the accompanying drawings shall be interpreted as illustrative only and not limiting. Changes in detail or structure may be made without departing from the present teachings. The foregoing description and following claims are intended to cover all such modifications and variations.

Various embodiments (examples) are described herein of various apparatuses, systems, and methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," "an embodiment," or the like, means that a particular feature, structure, or characteristic described in connection with the embodiment is included in at least one embodiment. Thus, appearances of the phrases "in various embodiments," "in some embodiments," "in one embodiment," "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

## Claims

1. A catheter (102, 202, 302, 402, 502, 602, 1002) for electrophysiological measurement, the catheter (102, 202, 302, 402, 502, 602, 1002) comprising:
a catheter shaft (104, 204, 1004) including a proximal end and a distal end; and
a flexible circuit (410, 510) disposed on the catheter shaft, the flexible circuit (410, 510) comprising:
a first dielectric layer (412, 512);
an electrical conductor (314, 414, 514) disposed on the first dielectric layer (412, 512);
a second dielectric layer (416, 516) disposed on the electrical conductor (414, 514); and
an electrode (108, 208, 408, 508, 608, 1008) coupled to the electrical conductor (314, 414, 514);
wherein the second dielectric layer (416, 516) includes one or more apertures (420, 520) to expose the electrode (108, 208, 408, 508, 608, 1008) to provide a contact surface area for placement against tissue within a body, wherein the contact surface area (A1, A2) of the electrode (108, 208, 408, 508, 608, 1008) includes an impedance reduction layer (418, 518) comprising a surface texture having a pattern and a surface roughness.

2. The catheter (102, 202, 302, 402, 502, 602, 1002) of claim 1, further comprising an intermediate layer (522) disposed on the electrical conductor conductor (314, 414, 514) and configured to facilitate adhesion between the electrical conductor (314, 414, 514) and the impedance reduction layer (418, 518).

3. The catheter (102, 202, 302, 402, 502, 602, 1002) of claim 1, wherein the electrical conductor (314, 414, 514) is a trace positioned along the flexible circuit (210, 410, 510, 610A-D).

4. The catheter (402, 502) of any one of claims 1 to 3, further comprising a gold layer (522) disposed between a copper layer of the electrical conductor (414, 514) and the impedance reduction layer (418, 518).

5. The catheter (402, 502) of claim 4, wherein the impedance reduction layer (418, 518) is configured to reduce the impedance at the electrode (408, 508) by at least fifty percent as compared to a similarly sized electrode without the impedance reduction layer (418, 518).

6. The catheter (102, 202, 302, 402, 502, 602, 1002) of any one of claims 1 to 5, wherein the impedance reduction layer (418, 518) has a thickness of between 1 µm and 30 µm, and/or wherein the impedance reduction layer (418, 518) is configured to increase the biocompatibility of the electrode (108, 208, 408, 508, 608, 1008).

7. The catheter (102, 202, 302, 402, 502, 602, 1002) of any one of claims 1 to 6, wherein the contact surface area (A1, A2) of the impedance reduction layer (418, 518) is 1.0 mm² or less, preferably 0.5 mm² or less.

8. The catheter (102, 202, 302, 402, 502, 602, 1002) of any one of claims 1 to 7, wherein the catheter is a planar catheter.

9. The catheter (102, 202, 302, 402, 502, 602, 1002) of any one of claims 1 to 7, wherein the catheter is a basket catheter.

10. The catheter (102, 202, 302, 402, 502, 602, 1002) of any one of claims 1 to 7, wherein the catheter is a linear catheter.

11. A method of making a catheter (102, 202, 302, 402, 502, 602, 1002) including a reduced impedance electrode (108, 208, 408, 508, 608, 1008), the method comprising:
disposing a flexible circuit (410, 510) on a catheter shaft (104, 204, 1004);
disposing a first dielectric layer (412, 512) on the flexible circuit (410, 510);
disposing an electrical conductor (314, 414, 514) on the first dielectric layer (412, 512);
disposing a second dielectric layer (416, 516) on the electrical conductor (414, 514);and
disposing an impedance reduction layer (418, 518) on a contact surface area (A1, A2) of the electrical conductor (102, 202, 302, 402, 502, 602, 1002);
wherein the second dielectric layer (416, 516) includes one or more apertures (420, 520) to expose the electrode (108, 208, 408, 508, 608, 1008) to provide a contact surface area for placement against tissue within a body, wherein the contact surface area (A1, A2) of the electrode (108, 208, 408, 508, 608, 1008) includes the impedance reduction layer (418, 518) comprising a surface texture having a pattern and a surface roughness.

12. The method of claim 10, further comprising disposing an intermediate layer (522) on the electrical conductor conductor (314, 414, 514), the intermediate layer (522) configured to facilitate adhesion between the electrical conductor (314, 414, 514) and the impedance reduction layer (418, 518).

13. The catheter (102, 202, 302, 402, 502, 602, 1002) of any one of claims 11 to 12, wherein the catheter is a planar catheter.

14. The catheter (102, 202, 302, 402, 502, 602, 1002) of any one of claims 11 to 12, wherein the catheter is a basket catheter.

15. The catheter (102, 202, 302, 402, 502, 602, 1002) of any one of claims 11 to 12, wherein the catheter is a linear catheter.
